# EUROPEAN PATENT SPECIFICATION

(11) **EP 1 523 289 B1**
(45) Date of publication and mention of the grant of the patent: **06.12.2006**
(21) Application number: 03748951.5
(22) Date of filing: 18.07.2003
(51) Int. Cl.: A61F 2/06

(54) **Stent coating holder**
Haltevorrichtung zur Beschichtung eines Stents
Support pour le revêtement d'une endoprothèse vasculaire

(30) Priority: 19.07.2002 US 198094
(43) Date of publication of application: 20.04.2005
(60) Divisional application: 06016108.0
(73) Proprietor: Boston Scientific Limited, St. Michael (BB)
(72) Inventor: EPSTEIN, Samuel, Watertown, MA 02472 (US); HANSEN, Henrik, 3751 Astermarie (DK); COYLE, Simon, Galway (IE); GRENHAM, Niall, Galway (IE); HAYES, Micheal, Galway (IE); SOBRINO SERRANO, Gabriel, Galway (IE)
(74) Representative: Altenburg, Udo
(86) International application number: PCT/US2003/022586
(87) International publication number: WO 2004/008995

(56) References cited:
- WO-A-03/045456
- US-A- 5 584 875
- US-B1- 6 527 863

## Description

### Field Of The Invention

The present invention generally regards the holding of stents during manufacture to enable the application of therapeutic and/or protective coatings. More specifically, the present invention provides stent holders that securely retain stents during the application of a coating while minimizing compressive and tensile forces applied to the stents and disruptions to the coating due to holder blockage of coating deposition.

### Background

Medical implants are used for innumerable medical purposes, including the reinforcement of recently re-enlarged lumens, the replacement of ruptured vessels, and the treatment of disease such as vascular disease by local pharmacotherapy, *i.e*., delivering therapeutic drug doses to target tissues while minimizing systemic side effects. Such localized delivery of therapeutic agents has been proposed or achieved using medical implants which both support a lumen within a patient's body and place appropriate coatings containing absorbable therapeutic agents at the implant location.

The term "therapeutic agent" as used herein includes one or more "therapeutic agents" or "drugs". The terms "therapeutic agents" and "drugs" are used interchangeably herein and include pharmaceutically active compounds, nucleic acids with and without carrier vectors such as lipids, compacting agents (such as histones), virus (such as adenovirus, andenoassociated virus, retrovirus, lentivirus and α-virus), polymers, hyaluronic acid, proteins, cells and the like, with or without targeting sequences.

Specific examples of therapeutic agents used in conjunction with the present invention include, for example, pharmaceutically active compounds, proteins, cells, oligonucleotides, ribozymes, anti-sense oligonucleotides, DNA compacting agents, gene/vector systems (i.e., any vehicle that allows for the uptake and expression of nucleic acids), nucleic acids (including, for example, recombinant nucleic acids; naked DNA, cDNA, RNA; genomic DNA, cDNA or RNA in a non-infectious vector or in a viral vector and which further may have attached peptide targeting sequences; antisense nucleic acid (RNA or DNA); and DNA chimeras which include gene sequences and encoding for ferry proteins such as membrane translocating sequences ("MTS") and herpes simplex virus-1 ("VP22")), and viral, liposomes and cationic and anionic polymers and neutral polymers that are selected from a number of types depending on the desired application. Non-limiting examples of virus vectors or vectors derived from viral sources include adenoviral vectors, herpes simplex vectors, papilloma vectors, adeno-associated vectors, retroviral vectors, and the like. Non-limiting examples of biologically active solutes include anti-thrombogenic agents such as heparin, heparin derivatives, urokinase, and PPACK (dextrophenylalanine proline arginine chloromethylketone); antioxidants such as probucol and retinoic acid; angiogenic and anti-angiogenic agents and factors; agents blocking smooth muscle cell proliferation such as rapamycin, angiopeptin, and monoclonal antibodies capable of blocking smooth muscle cell proliferation; anti-inflammatory agents such as dexamethasone, prednisolone, corticosterone, budesonide, estrogen, sulfasalazine, acetyl salicylic acid, and mesalamine; calcium entry blockers such as verapamil, diltiazem and nifedipine; antineoplastic / antiproliferative / anti-mitotic agents such as paclitaxel, 5-fluorouracil, methotrexate, doxorubicin, daunorubicin, cyclosporine, cisplatin, vinblastine, vincristine, epothilones, endostatin, angiostatin and thymidine kinase inhibitors; antimicrobials such as triclosan, cephalosporins, aminoglycosides, and nitorfurantoin; anesthetic agents such as lidocaine, bupivacaine, and ropivacaine; nitric oxide (NO) donors such as lisidomine, molsidomine, L-arginine, NO-protein adducts, NO-carbohydrate adducts, polymeric or oligomeric NO adducts; anti-coagulants such as D-Phe-Pro-Arg chloromethyl ketone, an RGD peptide-containing compound, heparin, antithrombin compounds, platelet receptor antagonists, anti-thrombin antibodies, anti-platelet receptor antibodies, enoxaparin, hirudin, Warafin sodium, Dicumarol, aspirin, prostaglandin inhibitors, platelet inhibitors and tick antiplatelet factors; vascular cell growth promotors such as growth factors, growth factor receptor antagonists, transcriptional activators, and translational promotors; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; agents which interfere with endogeneus vascoactive mechanisms; survival genes which protect against cell death, such as anti-apoptotic Bcl-2 family factors and Akt kinase; and combinations thereof. Cells can be of human origin (autologous or allogenic) or from an animal source (xenogeneic), genetically engineered if desired to deliver proteins of interest at the insertion site. Any modifications are routinely made by one skilled in the art.

Polynucleotide sequences useful in practice of the invention include DNA or RNA sequences having a therapeutic effect after being taken up by a cell. Examples of therapeutic polynucleotides include anti-sense DNA and RNA; DNA coding for an anti-sense RNA; or DNA coding for tRNA or rRNA to replace defective or deficient endogenous molecules. The polynucleotides can also code for therapeutic proteins or polypeptides. A polypeptide is understood to be any translation product of a polynucleotide regardless of size, and whether glycosylated or not. Therapeutic proteins and polypeptides include as a primary example, those proteins or polypeptides that can compensate for defective or deficient species in an animal, or those that act through toxic effects to limit or remove harmful cells from the body. In addition, the polypeptides or proteins that can be injected, or whose DNA can be incorporated, include without limitation, angiogenic factors and other molecules competent to induce angiogenesis, including acidic and basic fibroblast growth factors, vascular endothelial growth factor, hif-1, epidermal growth factor, transforming growth factor α and β, platelet-derived endothelial growth factor, platelet-derived growth factor, tumor necrosis factor α, hepatocyte growth factor and insulin like growth factor; growth factors; cell cycle. inhibitors including CDK inhibitors; anti-restenosis agents, including p15, p16, p18, p19, p21, p27, p53, p57, Rb, nFkB and E2F decoys, thymidine kinase ("TK") and combinations thereof and other agents useful for interfering with cell proliferation, including agents for treating malignancies; and combinations thereof. Still other useful factors, which can be provided as polypeptides or as DNA encoding these polypeptides, include monocyte chemoattractant protein ("MCP-1"), and the family of bone morphogenic proteins ("BMP's"). The known proteins include BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively or, in addition, molecules capable of inducing an upstream or downstream effect of a BMP can be provided. Such molecules include any of the "hedgehog" proteins, or the DNA's encoding them.

The delivery of expandable stents is a specific example of a medical procedure that involves the deployment of coated implants. Expandable stents are tube-like medical devices, typically made from stainless steel, Tantalum, Platinum or Nitinol alloys, designed to be placed within the inner walls of a lumen within the body of a patient. These stents are typically maneuvered to a desired location within a lumen of the patient's body and then expanded to provide internal support for the lumen. The stents may be self-expanding or, alternatively, may require external forces to expand them, such as by inflating a balloon attached to the distal end of the stent delivery catheter.

Because of the direct contact of the stent with the inner walls of the lumen, stents have been coated with various compounds and therapeutic agent s to enhance their effectiveness. These coatings may, among other things, be designed to facilitate the acceptance of the stent into its applied surroundings. Such coatings may also be designed to facilitate the delivery of one of the foregoing therapeutic agents to the target site for treating, preventing, or otherwise affecting the course of a disease or tissue or organ dysfunction.

Where the stent has been coated, care must be taken during its manufacture and delivery within the patient to ensure the coating is evenly applied and firmly adherent to the stent, and further that the coating is not damaged or completely removed from the implant during the deployment process. When the amount of coating is depleted the implant's effectiveness may be compromised and additional risks may be inured into the procedure. For example, when the coating of the implant includes a therapeutic, if some of the coating were removed during deployment, the therapeutic may no longer be able to be administered to the target site in a uniform and homogenous manner. Thus, some areas of the target site may receive high quantities of therapeutic while others may receive low quantities of therapeutic. Similarly, if the therapeutic is ripped from the implant it can reduce or slow down the blood flowing past it, thereby, increasing the threat of thrombosis or, if it becomes dislodged, the risk of embolisms. In certain circumstances, the removal and reinsertion of the stent through a second medical procedure may be required where the coatings have been damaged or are defective.

The mechanical process of applying a coating onto a stent may be accomplished in a variety of ways, including, for example, the spraying of the coating substance onto the stent and so-called spin-dipping, *i.e.,* dipping a spinning stent into a coating solution to achieve the desired coating. Common to these processes is the need to securely hold the stent in a desired orientation during the process of coating application to ensure an intact, robust coating of the desired thickness is formed on the stent.

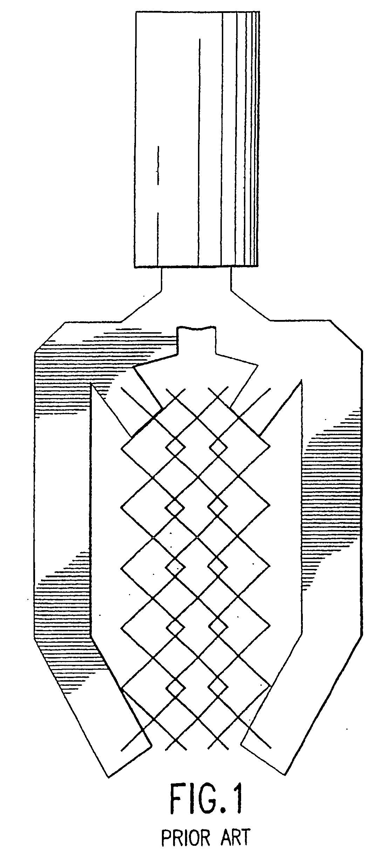

If the stent is held too loosely, it may either shift during the coating process or it may become prematurely separated from the holder, resulting in an inconsistent or damaged coating. For example, Fig. 1 illustrates a prior art spin-dipping stent holder 1 with a grappling clip bent in a manner that allows the stent to be maneuvered into the grappling clip's grasp. Difficulties with properly aligning the stent on this device, high centripetal forces generated during spinning, and low retention forces on the stent can result in premature separation of the stent from the holder. Further, this device is not is suitable for universal use across a range of stent sizes, as it must be custom built for each specific stent size.

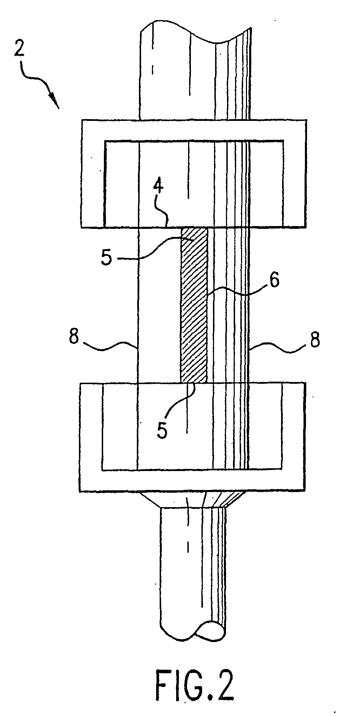

On the other hand, if the stent is held with too great a compressive or tensile force, it and/or its supporting structure may buckle, collapse or prematurely expand. For example, Fig. 2 illustrates another prior art stent holder 2 in which two tensioned cross wires 4 compress the stent ends 5 to hold stent 6 in place during coating spray application. Due to the need to generate substantial compression force on the stent to hold it in place, there occur problems such as mid-section bulging or buckling of longer stents, stent misalignment and accelerated wear and slippage of the wires and bushings used to secure the wires to the holder (not shown). In addition, due to the relatively large tensile loading, the support struts 8 must be made sufficiently large to avoid failure, which in turn can result in inadequate coating formation on the stent due to spray "shadowing," *i.e.,* incomplete coating spray application onto the stent due to structural elements blocking the spray. An additional disadvantages of this type of stent holder is its relatively high expense given its complexity and the need to use high strength materials.

US-A-5 584 875, which represents closest prior art, discloses a wire or a mandrel for holding a graft or a tube to be coated.

Thus, there is a need for a relatively inexpensive, robust stent holder which can positively locate and retain a stent during stent coating processes such as spin-dipping and spray coating, while not interfering with the application of the coating.

### Summary Of The Invention

The present invention is directed to an apparatus and method for overcoming the foregoing disadvantages. This is achieved according to the invention by a stent holder as defined in claim 1 and by a method as defined in claim 10. Advantageous embodiments of the stent holder and the method are defined in dependent claims 2 to 9 and 11. Specifically, there is provided an embodiment of a stent coating holder comprising an internal welded cross wire holder in which a wire with a loop at each end passes through the center of a stent and is held at both ends by loop holders. The loop holders spread their respective wire loops apart such that the loop contacts the inside edge of its respective end of the stent at two diametrically opposed points. The loop holders simultaneously maintain sufficient tension on the wire loops to generate a relatively light compressive force on the stent to positively locate it between the loop holders. Due to the light compression force and the location of the cross wire within the stent, the stent holder does not apply damaging forces to the stent, and minimizes the creation of spray shadows. Moreover, due to the wire loops' flexibility, the wire loops and holders can accommodate a range of stent lengths and diameters before a larger or smaller wire loop and holder is needed.

### Brief Description Of The Drawings

Fig. 1 is an illustration of a prior art spin-dipping stent coating holder.
Fig. 2 is an illustration of a prior art spray deposition stent coating holder.
Fig. 3 is an oblique view of a first embodiment of a stent coating holder in accordance with the present invention.
Fig. 4 is a side view of the wire of the first embodiment of a stent coating holder in accordance with the present invention.
Fig. 5 is a detail side view of the arrangement of the wire loop sides within a stent held in the first embodiment of a stent coating holder in accordance with the present invention.

### Detailed Description

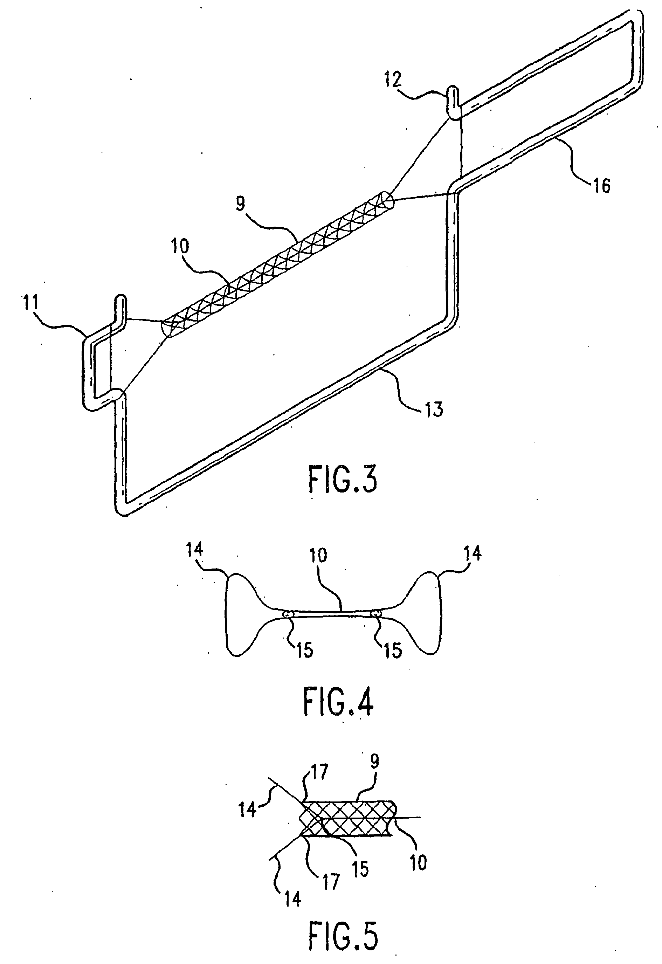

The present invention is directed to an apparatus and method for overcoming the foregoing disadvantages. Specifically, there shown in Fig. 3 a first embodiment of a stent coating holder in accordance with the present invention. As shown in Fig. 3, stent 9 is suspended on stent holding wire 10. Wire 10 passes through the center of stent 9 and is secured to two loop holders 11 and 12 at opposing ends of stent holding frame 13.

Stent holding wire 10 in this embodiment is formed as shown in Fig. 4. Each end of wire 10 is formed into a loop 14, with the end of each loop secured to wire 10 at a position 15 along the wire that results in a loop of a specific desired size. Stent holding wire 10 is formed from a material possessing sufficient tensile strength to support the stent during stent coating, and possessing sufficient elasticity to permit stent holding wire 10 to be readily formed into loops at its opposing ends of wire 10 and to permit a loop 14 to be collapsed in order to be inserted through the center of stent 9 when preparing to place the stent on the stent coating holder. In this embodiment, stent holding wire 10 is preferably formed from stainless steel wire with a diameter of 0,05 mm (0.002 inches), and the loops 14 are secured to wire 10 by welding.

Stent holding frame 13 is formed by bending a rod into the desired shape. The rod material should have sufficient resistance to bending such that frame 13 will be able to maintain adequate axial tension on stent holding wire 10 to securely retain the stent thereon, yet not be so stiff as to prevent the securing of the wire loops 14 over loop holders 11 and 12. The rod material must also be sufficiently resistant to bending to ensure that loop holders 11 and 12 will be able to keep their respective wire loops 14 adequately spread apart.

In this embodiment, the holder is preferably constructed from a 1.0 mm diameter stainless steel rod press-formed into the desired shape. As shown in Fig. 3, at one end of the rod, loop holder 11 is formed with sufficient height to securely retain one of the loops 14 on stent holding wire 10. Similarly, at the other end of stent holding frame 13, the other loop holder 12 also is formed from the rod with sufficient height to retain the second loop 14 on stent holding wire 10. Further, as illustrated in Fig. 3, the height of loop holder 12 may be extended a sufficient distance to form a handle 16.

Fig. 5 illustrates the arrangement of stent holding wire 10 at an end of a stent held in the holder. Stent holding wire 10 passes through the center of stent 9. The size of loop 14 and the length of stent holding wire 10 have been selected to ensure that the welded joint at position 15 remains far enough within the center of stent 9, and that the sides of loop 14 are spread by its loop holder (not shown) far enough to ensure the loop sides contact the inside edge of the end of stent 9 at diametrically-opposed positions 17.

The stent is mounted on the stent holder by first passing one of the loops 14 of stent holding wire 10 through the center of stent 9 until a portion of both loops 14 protrude from their respective ends of stent 9, and then one loop 14 is passed over a loop holder 11 or 12. Alternatively, one of the loops 14 may be first passed over a loop holder, then the other loop 14 may be passed through the center of stent 9. If handle 16 has been formed in stent holding frame 13, the first loop should be passed over loop holder 12. The remaining loop holder 11 is then gently pushed toward loop holder 12 far enough to permit the remaining free loop 14 to be passed over loop holder 11. When loop holder 11 is then released, the stent holding frame 13 acts as a spring to apply tension to the ends of stent holding wire 10 through loop holders 11 and 12, and the forces applied by the sides of loops 14 to their respective ends of stent 9 securely locate the stent both axially and transversely between loop holders 11 and 12.

The foregoing embodiment of the present invention provides a stent coating holder which: securely holds a stent without the need to exert large compressive or tensile forces, thereby minimizing the risk of stent deformation or misalignment due to application of excessive retaining or positioning forces; is relatively inexpensive to produce; may be readily adapted to be used with a range of stent lengths and diameters; minimizes coating spray shadows; and eliminates the need for any of the frequently-replaced wire securement bushings used in the prior art. This embodiment is also amenable to use in automatic loading machines for automation of the stent loading and coating process.

In addition to the foregoing embodiment, the present invention encompasses methods of use of the apparatus of the invention. The method of use of the first embodiment of the present invention comprises the steps of passing an end of a center wire of desired length through the center of a stent to which a coating is to be applied, placing a loop of a desired size formed at each end of the center wire over loop holders on opposing ends of a stent coating holder such that tension is applied to the center wire and the loop holders cause the sides of each loop to contact an end edge of the loops' respective ends of the stent, and applying a desired coating to the stent loaded on the stent coating holder. There is no required order for the steps of passing the wire through the stent and placing a first loop over a loop holder on the stent coating holder.

## Claims

1. A stent holder for holding a stent during application of a stent coating, **characterised in that** said holder comprises:
a center element (10) with a first flexible loop (14) at a first end and a second flexible loop (14) at a second end, wherein at least one of the first loop and the second loop can be elongated to pass through the stent, and further wherein a distance between the first loop and the second loop is less than a length of the stent; and
a stent holding frame (13) configured to hold the center element with the stent thereon under tension between a first loop holder (11) holding the first loop at a first end of the frame and a second loop holder (12) holding the second loop at a second end of the frame,
wherein a distance between the first loop holder and the second loop holder is greater than the length of the stent, and further wherein the first loop holder spreads the first loop wide enough in a direction transverse to a longitudinal axis of the stent that the first loop contacts a first end of the stent at two contact points (17), and the second loop holder spreads the second loop wide enough in a direction tranverse to the longitudinal axis of the stent that the second loop contacts a second end of the stent at two contact points.

2. The stent holder of claim 1, wherein the center element is one of a rod, a tube and a wire.

3. The stent holder of claim 1, wherein the center element is a wire and the first loop is formed by looping the first end of the wire back upon itself and affixing the first end of the wire to a position along the wire corresponding to a desired loop size.

4. The stent holder of claim 1, wherein the center element is an elongated continuous wire loop, and the first loop and the second loop are formed by twisting the elongated continuous wire loop until two sides of the loop contact one another, and affixing the sides of the elongated continuous wire loop to one another at the contact point.

5. The stent holder of claim 4, wherein the two sides of the elongated continuous wire loop are affixed to one another by welding.

6. The stent holder of claim 1, wherein the center element is an elongated continuous wire loop, and the first loop is formed by affixing two sides of the elongated continuous wire loop to one another at a first location corresponding to a desired size of the first loop, the second loop is formed by affixing the two sides of the elongated continuous wire loop at a second location corresponding to a desired size of the second loop, and wherein the portions of each of the two sides of the elongated continuous wire loop between the first position and the second position are substantially equal in length.

7. The stent holder of claim 6, wherein the two sides of the elongated continuous wire loop are affixed to one another by welding.

8. The stent holder of claim 7, wherein the loop holders are integrally formed with the stent holding frame.

9. The stent holder of claim 8, wherein the first loop holder and the second loop holder are formed in polygonal shapes and project away from one another in a plane containing the center element.

10. A method for using the stent coating holder of claim 1, comprising the steps of:
passing the first flexible loop of the center element through the center of the stent;
engaging the first loop on the first loop holder on the stent holding frame;
engaging the second flexible loop of the center element on the second loop holder on the stent holding frame, thereby placing the center element under tension and causing the first loop to contact the first end of the stent at two contact points and the second loop to contact the second end of the stent at two contact points; and
applying the stent coating to the stent.

11. The method of claim 10, wherein the center element is a wire, the first loop holder and the second loop holder are integrally formed with the stent holding frame and project away from one another in a plane containing the center wire, and the steps of engaging the first loop on the first loop holder and engaging the second loop on the second loop holder comprise the steps of:
placing the first loop over the projection of the first loop holder; and
placing the second loop over the projection of the second loop holder, thereby placing the center wire under tension and causing the first loop to contact the first end of the stent at two contact points and the second loop to contact the second end of the stent at two contact points.

## Patentansprüche

1. Eine Stent-Halterung zum Halten eines Stents während der Aufbringung einer Stentbeschichtung, **dadurch gekennzeichnet** das die Halterung umfasst:
ein mittig gelegenes Element (10) mit einer ersten flexiblen Schleife (14) an einem ersten Ende und einer zweiten flexiblen Schleife (14) an einem zweiten Ende, wobei mindestens eine der ersten Schleife und der zweiten Schleife gestreckt werden kann, sodass sie durch den Stent passiert, und wobei ein Abstand zwischen der ersten Schleife und der zweiten Schleife weniger als die Länge des Stents beträgt; und
ein Halterahmen für den Stent (13), der konfiguriert ist, um das mittig gelegene Element mit dem darauf befindlichen Stent unter Spannung zwischen einer ersten Schleifenhalterung (11) die die erste Schleife an einem ersten Ende des Rahmens hält, und einer zweiten Schleifenhalterung (12), die die zweite Schleife an einem zweiten Ende des Rahmens hält, zu halten,
wobei eine Distanz zwischen der ersten Schleifenhalterung und der zweiten Schleifenhalterung größer ist als die Länge des Stents und wobei weiter die erste Schleifenhalterung die erste Schleife in einer quer zu einer longitudinalen Achse des Stents gelegenen Richtung breit genug spreizt, dass die erste Schleife ein erstes Ende des Stents an mindestens zwei Kontaktpunkten (17) kontaktiert und die zweite Schleifenhalterung die zweite Schleife breit genug in einer quer zu der longitudinalen Achse des Stents liegenden Richtung spreizt, sodass die zweite Schleife ein zweites Ende des Stents an zwei Kontaktpunkten kontaktiert.

2. Stent-Halterung gemäß Anspruch 1, worin das mittig gelegene Element unter einem Stab, einem Rohr und einem Draht gewählt ist.

3. Stent-Halterung gemäß Anspruch 1, wobei das mittig gelegene Element ein Draht ist und die erste Schleife gebildet wird, indem das erste Ende des Drahts auf sich selbst zurück gebogen wird und das erste Ende des Drahts an einer Position entlang des Drahts, die einer gewünschten Schleifengröße entspricht, befestigt wird.

4. Stent-Halterung gemäß Anspruch 1, worin das mittig gelegene Element eine gestreckte kontinuierliche Drahtschleife ist und die erste Schleife und die zweite Schleife gebildet werden, indem die gestreckte kontinuierliche Drahtschleife verdreht wird, bis zwei Seiten der Schleife miteinander in Kontakt stehen und die Seiten der gestreckten kontinuierlichen Drahtschleife an dem Kontaktpunkt aneinander befestigt werden.

5. Stent-Halterung gemäß Anspruch 4, wobei die zwei Seiten der gestreckten kontinuierlichen Drahtschleife aneinander durch Schweißen befestigt werden.

6. Stent-Halterung gemäß Anspruch 1, wobei das mittig gelegene Element eine gestreckte kontinuierliche Drahtschleife ist und die erste Schleife gebildet wird, indem zwei Seiten der gestreckten kontinuierlichen Drahtschleife aneinander an einer ersten Stelle, die der gewünschten Größe der ersten Schleife entspricht, befestigt werden und die zweite Schleife gebildet wird, indem die zwei Seiten der gestreckten kontinuierlichen Drahtschleife an einem zweiten Punkt, der einer gewünschten Größe der zweiten Schleife entspricht, befestigt ist, und wobei die Anteile jeder der zwei Seiten der gestreckten kontinuierlichen Drahtschleife zwischen der ersten Position und der zweiten Position im Wesentlichen gleicher Länge sind.

7. Stent-Halterung gemäß Anspruch 6, wobei die zwei Seiten der gestreckten kontinuierlichen Drahtschleife aneinander durch Schweißen befestigt sind.

8. Stent-Halterung gemäß Anspruch 7, wobei die Schleifenhalterungen integral gebildet sind mit dem Stent-Halterahmen.

9. Stent-Halterung gemäß Anspruch 8, wobei die erste Schleifenhalterung und die zweite Schleifenhalterung zu polygonalen Formen gebildet sind und voneinander in einer Ebene, die das mittig gelegene Element enthält, wegzeigen.

10. Ein Verfahren zur Verwendung der Stentbeschichtungshalterung gemäß Anspruch 1 umfassend die Schritte:
passieren der ersten flexiblen Schleife des mittig gelegenen Elements durch die Mitte des Stents;
Befestigung der ersten Schleife auf der ersten Schleifenhalterung des Stent-Halterahmens;
Befestigung der zweiten flexiblen Schleife des mittig gelegenen Elements auf der zweiten Schleifenhalterung des Stent-Halterahmens, wodurch das mittig gelegene Element unter Spannung gestellt wird und ein Kontakt der ersten Schleife an dem ersten Ende des Stents an zwei Kontaktpunkten und ein Kontakt der zweiten Schleife an dem zweiten Ende des Stents an zwei Kontaktpunkten hergestellt wird; und
die Stentbeschichtung auf den Stent aufgebracht wird.

11. Verfahren gemäß Anspruch 10, wobei das mittig gelegene Element ein Draht ist, die erste Schleifenhalterung und die zweite Schleifenhalterung integraler Bestandteil des Stent-Halterahmens ist und voneinander in einer Ebene, die den mittig gelegenen Draht enthält, wegzeigen, und die Schritte der Befestigung der ersten Schleife auf dem ersten Schleifenhalter und der Befestigung der zweite Schleife auf dem zweiten Schleifenhalter die Schritte umfassend:
platzieren der ersten Schleife über den Vorsprung auf dem Schleifenhalter; und
platzieren der zweiten Schleife über den Vorsprung auf dem zweiten Schleifenhalter, wodurch das mittig gelegene Element unter Spannung gestellt wird und ein Kontakt der ersten Schleife an dem ersten Ende des Stents an zwei Kontaktpunkten und ein Kontakt der zweiten Schleife an dem zweiten Ende des Stents an zwei Kontaktpunkten hergestellt wird.

## Revendications

1. Dispositif de maintien d'une endoprothèse vasculaire pour le maintien d'une endoprothèse au cours de l'application d'un revêtement d'endoprothèse vasculaire, **caractérisé en ce que** ledit dispositif de maintien comprend :
un élément central (10) avec une première boucle flexible (14) à une première extrémité et une seconde boucle flexible (14) à une seconde extrémité, dans lequel au moins une de la première boucle et de la seconde boucle peut être allongée pour traverser l'endoprothèse vasculaire, et en outre dans lequel une distance entre la première boucle et la seconde boucle est inférieure à une longueur de l'endoprothèse vasculaire ; et
un cadre (13) de maintien de l'endoprothèse vasculaire configuré pour maintenir l'élément central avec l'endoprothèse sur celui-ci sous tension entre un dispositif de maintien d'une première boucle (11) maintenant la première boucle à une première extrémité du cadre et un dispositif de maintien d'une seconde boucle (12) maintenant la seconde boucle à une seconde extrémité du cadre,
dans lequel une distance entre le dispositif de maintien de la première boucle et le dispositif de maintien de la seconde boucle est supérieur à la longueur de l'endoprothèse vasculaire, et en outre dans lequel le dispositif de maintien de la première boucle étend la première boucle suffisamment largement en une direction transversale à un axe longitudinal de l'endoprothèse vasculaire afin que la première boucle entre en contact avec une première extrémité de l'endoprothèse vasculaire en deux points de contact (17), et le dispositif de maintien de la seconde boucle étend la seconde boucle suffisamment largement en une direction transversale à l'axe longitudinal de l'endoprotrèse vasculaire de manière à ce que la seconde boucle entre en contact avec une seconde extrémité de l'endoprothèse vasculaire en deux points de contact.

2. Dispositif de maintien d'une endoprothèse vasculaire selon la revendication 1, dans lequel l'élément central est l'un parmi une tige, un tube et un fil.

3. Dispositif de maintien d'une endoprothèse vasculaire selon la revendication 1, dans lequel l'élément central est un fil et la première boucle est formée en faisant faire une boucle à la première extrémité du fil sur lui-même et en fixant la première extrémité du fil en une position le long du fil correspondant à une taille de boucle souhaitée.

4. Dispositif de maintien d'une endoprothèse vasculaire selon la revendication 1, dans lequel l'élément central est une boucle de fil continu allongé, et la première boucle et la seconde boucle sont formées en tordant la boucle de fil continu allongé jusqu'à ce que deux cotés de la boucle entrent en contact l'un avec l'autre, et en fixant les cotés de la boucle de fil continu allongé l'un à l'autre au niveau du point de contact.

5. Dispositif de maintien d'une endoprothèse vasculaire selon la revendication 4, dans lequel les deux cotés de la boucle de fil continu allongé sont fixés l'un à l'autre par soudure.

6. Dispositif de maintien d'une endoprothèse vasculaire selon la revendication 1, dans lequel l'élément central est une boucle de fil continu allongé, et la première boucle est formée par fixation de deux cotés de la boucle de fil continu allongé l'un à l'autre en un premier emplacement correspondant à une taille souhaitée de la première boucle, la seconde boucle est formée par fixation des deux cotés de la boucle de fil continu allongé en un second emplacement correspondant à une taille souhaitée de la seconde boucle, et dans lequel les parties de chacun des deux cotés de la boucle de fil continu allongé entre la première position et la seconde position sont sensiblement égales en longueur.

7. Dispositif de maintien d'une endoprothèse vasculaire selon la revendication 6, dans lequel les deux cotés de la boucle de fil continu allongé sont fixés l'un à l'autre par soudure.

8. Dispositif de maintien d'une endoprothèse vasculaire selon la revendication 7, dans lequel les dispositifs de maintien d'ure boucle sont formés de manière solidaire avec le cadre de maintien de l'endoprothèse vasculaire.

9. Dispositif de maintien d'une endoprothèse vasculaire selon la revendication 8, dans lequel le dispositif de maintien de la première boucle et le dispositif de maintien de la seconde boucle sont formés en ces formes polygonales et se projettera de manière éloignée l'un de l'autre dans un plan contenant l'élément central.

10. Procédé d'utilisation d'un dispositif de maintien pour le revêtement d'une endoprothèse vasculaire selon la revendication 1, comprenant les étapes consistant à :
passer la première boucle flexible de l'élément central au travers du centre de l'endoprothèse vasculaire ;
engager la première boucle sur le dispositif de maintien de la première boucle sur le cadre de maintien de l'endoprothèse vasculaire ;
engager la seconde boucle flexible de l'élément central sur le dispositif de maintien de la seconde boucle sur le cadre de maintien de l'endoprothèse vasculaire, plaçant ainsi l'élément central sous tension et provoquant le contact de la première boucle avec la première extrémité de l'endoprothèse vasculaire en deux points de contact et le contact de la seconde boucle avec la seconde extrémité de l'endoprothèse vasculaire en deux points de contact ; et
appliquer le revêtement d'endoprothèse vasculaire à l'endoprothèse vasculaire.

11. Procédé selon la revendication 10, dans lequel l'élément central est un fil, le dispositif de maintien de la première boucle et le dispositif de maintien de la seconde boucle sont formés de manière solidaire avec le cadre de maintien de l'endoprothèse vasculaire et se projettent de manière éloignée l'un de l'autre dans un plan contenant le fil central, et les étapes consistant à engager la première boucle sur le dispositif de maintien de la première boucle et à engager la seconde boucle sur le dispositif de maintien de la seconde boucle comprennent les étapes consistant à :
placer la première boucle sur la projection du dispositif de maintien de la première boucle ; et
placer la seconde boucle sur la projection du dispositif de maintien de la seconde boucle, plaçant ainsi le fil central sous tension et provoquant le contact de la première boucle avec la première extrémité de l'endoprothèse vasculaire en deux points de contact et le contact de la seconde boucle avec la seconde extrémité de l'endoprothèse vasculaire en deux points de contact.
